Europäisches Patentamt

European Patent Office

Office européen des brevets

(19)

(11) Publication number: **0 104 881**
A2

(12) # EUROPEAN PATENT APPLICATION

(21) Application number: **83305573.4**

(22) Date of filing: **21.09.83**

(51) Int. Cl.³: **G 01 N 33/80**

(30) Priority: **22.09.82 US 421227**

(43) Date of publication of application:
**04.04.84 Bulletin 84/14**

(84) Designated Contracting States:
**AT BE CH DE FR GB IT LI LU NL SE**

(71) Applicant: **ORTHO DIAGNOSTIC SYSTEMS INC.**
**One Johnson & Johnson Plaza**
**New Brunswick New Jersey 08933(US)**

(72) Inventor: **Falkowski, Frank**
**RD 1**
**Lebanon New Jersey(US)**

(72) Inventor: **Savitz, Steven**
**1300 Alicia Avenue**
**Teaneck New Jersey 07666(US)**

(72) Inventor: **Graham, Henry, Jr.**
**RD 2 Box 74 Sand Hill Road**
**Annandale New Jersey 08801(US)**

(74) Representative: **Jones, Alan John et al,**
**CARPMAELS & RANSFORD 43 Bloomsbury Square**
**London, WC1A 2RA(GB)**

(54) **Test kit for ABO transfusion compatability.**

(57) Apparatus for bedside use for eliminating fatal cross-matching transfusion errors. The preferred embodiment provides a housing having antisera reactive with the antigens present on the surface of the erythrocytes of the donor blood. The preferred housing has removably mounted therein a lancet for obtaining the patient's blood as well as a porous plastic member provided for blood pick up and serving as a reaction support vehicle. The objective determination of the presence or virtual absence of red color in an observation area of the porous plastic member permits facile determination of a go/no go transfusion situation.

FIG-2

Croydon Printing Company Ltd.

## TEST KIT FOR ABO TRANSFUSION COMPATABILITY

### Field of the Invention

This invention relates to the field of blood banking in general and specifically, relates to patient bedside apparatus designed to virtually eliminate lethal tranfusion errors.

### Background of the Invention

Tranfusions of life giving blood in the hospital have become so numerous as to be commonplace. For instance, during 1978 it has been estimated that approximately 9.4 million units of blood were transfused. See Transfusion-Associated Fatalities: Review of Bureau of Biologics Reports 1976-1978, Transfusion, Vol. 20, No. 6, p. 653-661 (Nov.-Dec. 1980). During a concurrent three year period, however, 64 transfusion-associated fatalities were documented providing an estimated mortality rate of approximately 1 per 422,000 units transfused. Deaths were held primarily attributable to hemolytic transfusion reactions wherein the recipient's antisera reacted with antigenic determinants present on the surface of the red blood cells of the donor unit. These antigenic determinants are the same ones that commonly identify blood types such as A, B, AB, or O. The latter group, or universal donor group, is unique in that it does not have either A or B antigens associated with the red blood cells. In contrast, a type A person is one who has A type antigens on the surface of red blood cells. Such a person must therefore lack anti-A sera in his blood stream but can be expected to have anti-B sera. As a result, the A-type person can only receive blood cells which do not have B antigens present thereon. It follows therefore that an A type person may safely receive either A or O type blood.

Should the A type person be provided with B type blood or AB type blood, either of which contain B antigens on the surface of the red blood cells, such cells can be expected to react with the anti-B sera present in the A type individual's blood stream. This reaction, the so-called a hemolytic transfusion reaction, is of such a serious nature that it may be lethal if the volume of transfused blood is large enough. The same situation similarly holds for B and AB blood type individuals. It is thus clear that compatibility between A, B, AB and O type (ABO type) individuals is of extreme importance.

Transfusion-related deaths due to ABO incompatability have typically been associated with either errors in clinical laboratory procedures identifying the unit of blood or, more commonly with clerical errors in the labelling of the patient and very occasionally of the donor bags. Perhaps most common are the errors generated in the hospital environment due to patient relocations or patient misidentifications all of which are difficult to control. See for instance the article by Schmidt et al. entitled "Sources of Error in a Hospital Blood Bank", Transfusion, Vol. 3:198-201 (1963). For a more rigorous treatment of the symptoms of hemolytic transfusion errors refer to Pineda et al., "Hemolytic Transfusion Reaction", Mayo Clin. Proc. 53:378-390, 1978. The manner in which identification errors can occur from the time of admission to transfusion of the patient has also been reviewed by Guy, L. R., "Transfusion Patient Identification and Related Problems", Laboratory Medicine, Vol. 12, No. 9:542-545 (September, 1981).

Clearly, if such lethal errors due to the mismatching of donor type with recipient type are to be avoided, some 'last minute', fail-safe method must be instituted which is economical and easily performed. Conventional

ORD-44

approaches to lethal error avoidance have employed two types of methods. The first approach is directed towards the development of a computer system capable of reducing clerical discrepancies such as the system described by Grindon and Liles in Transfusion, Vol. 21, No. 2:199-202 (March-April, 1981) in an article entitled "Center Error Reduction by Recheck of Blood Type". A computer oriented system is, however, highly susceptible to input errors by typists and other data key-in related problems. The computer approach is further disadvantageous as well as just generally being unwieldy and nonconducive to rapid employment.

Another generic approach has employed a full typing system for example the IDENTITEST (available from Ortho Diagnostics G.m.b.H. Germany). IDENTITEST" is a slide/cell test performed on both the donor and the recipient in accordance with German statutory requirements. This bedside test essentially duplicates the previously performed, laboratory typing procedures conducted on both the donor blood as well as that of the patient. The test disadvantageously requires multiple reagent bottles including anti-A, anti-B, anti-AB, and anti-D as well as a paper slide for providing a permanent record. A similar test has been developed by the Nordisk Insulinlaboratorium, from Denmark and is called the Eldon Card. The Eldon Card, although also a paper slide test, relies on the whole blood sample to dissolve antibodies previously dried on the paper slide. Consequently, the end point strength of the anti-sera reactions is weak and requires experienced subjective analysis.

It is an object of the present invention to obviate the need to do a full typing regimen and to provide a test capable of objective analysis whereby only those errors which would result in fatalities are eliminated.

Still another technique has been described by Anderson in an article entitled "Analytical Techniques for Cell Fractions, Use of Cellulose Wicks to Monitor Agglutination Reactions", Analytical Biochemistry, 38, 175-189 (1970). Anderson described a method of ABO typing wherein Whatman paper wicks were inserted into plastic wells having mixed therein the blood sample and typing serum. The wells were previously rocked at room temperature prior to the addition of the paper wick. Strong agglutination reactions in the Anderson system result in limited movement of the red blood cells whereas nonagglutinated systems permit relatively unhindered movement. Anderson's system, however, suffers from the relative difficulty of controlling paper-wick porosity. Consequently, fluid movement, especially critical in weakly reactive blood types such as the AB subgroups can easily go undetected and undiagnosed. Additionally, the Anderson system is difficult to employ at the bedside environment where it is most needed. This weakness, coupled with the often subjective evaluation required, severly limits its usefulness.

It is an object of the present invention to provide an ABO compatability test system which, although relying on capillarity as the Anderson system, is simpler to use in that the reactions are objectively clear and provide a go/ no go determination. It is a further object of the present invention to provide a test kit which relies on the relatively high degree of accuracy of blood unit indentification and tests instead, the recipient's compatability with the blood unit rather than performing a full blood typing procedure.

Summary of the Invention

In accordance with the principles and objects of the present invention, there are provided bedside kits for testing the ABO compatability of a transfusion recipient to a labelled blood unit. The kit, in its most preferred embodiment, comprises a housing which is attachable to the blood unit bag, which housing has incorporated therein a disposable lancet for obtaining a small blood sample from the recipient. Also incorporated in the housing is a sealed well of preferably sterile antisera of the type reactive with the blood of the donor bag. The well is ideally unsealed only a moment just prior to use. The housing lastly incorporates a porous plastic member, preferably mounted within a holder for easy manipulation, all of which may be removed from the housing. The plastic member is dimensioned appropriately to, by capillary action, pick up a specified quantity of the recipient's blood. The plastic member is then dipped into the well containing the antisera so that the antisera is also 'wicked' into the same physical location as the recipient's blood. If the patient has been properly identified (and the donor type is correct as assumed), the antisera will agglutinate the recipient's blood thereby prohibiting its further upward movement in the porous plastic member by capillary action. The preferred embodiment of the antisera reagent will have additionally incorporated therein a dye which remains free to migrate by capillary action in the agglutinated case to become visible. Should there be an incorrect identification of the patient, and a resulting absence of agglutination, such dye will be masked by the presence of red cells which, by their nonagglutinated nature, are also capable of movement by capillary action. This presence of red cells, seen as a red color at or near the top of the plastic member, serves as an indication to stop the transfusion and correct the source of error. If the dye within the anti-sera is, for instance, of a green color, then clearly an objective red-no-go/green-go criteria is established whereby the safety of the transfused recipient is ensured.

)-44

## Brief Description of the Drawings

Figure 1 is a side view of the porous plastic member;

Figure 2 is a side view of one embodiment of the testing kit employing the porous plastic member of Figure 1;

Figure 3 is a side view of the embodiment of Figure 2 as it appears following use;

Figure 4 is a perspective view of an alternative embodiment of the bedside kit;

Figure 5 is a perspective view of the porous plastic member and well of the Figure 4 embodiment;

Figure 6 is a perspective view of the Figure 4 embodiment following use;

Figure 7 is a perspective view of the preferred embodiment;

Figure 8 is a perspective view of an intermediate step in use of the preferred embodiment of Figure 7;

Figure 9 is a perspective view of the preferred embodiment of Figure 7 following use;

Figure 10 is a perspective "see-through" view of another alternative embodiment prior to use;

Figure 11 is a perspective view of the embodiment of Figure 10 following use; and

Figure 12 is a perspective view of yet another alternative embodiment.

ORD-44

Detailed Description of the Drawings and Preferred Embodiment

According to the principles of the present invention, all of the embodiments, including the preferred embodiments, rely on the presumption that the donor blood bag containing the blood to be transfused to the recipient has been correctly labelled. Studies referred to earlier have demonstrated this assumption to be statistically valid. Further, the present invention is grounded upon the test logic that in order to avoid lethal errors, it need only be shown that the recipient patient's cells have the same antigens as those of the cells in the donor bag. The fact that the patient's cells may have additional antigens for instance in the case of an AB type patient receiving A type blood is, for the purposes of avoiding lethal errors, relatively unimportant. Since approximately 45% of the human population is of type A and 10% is of type B, only 1 test becomes necessary approximately 55% of the time. Approximately 45% of the human population is type O and therefore all of these persons are universal donors and no test is required since hemolytic transfusion type lethal errors do not occur with donative blood of this type. Consequently, it is only when the donor blood is of type AB will a need arise to test the patient for the presence of both A and B antigens on the patient's blood cells. Only in this very limited circumstance will two tests be required, however, for the most part, this burden will be tempered by the fact that many hospitals preferentially avoid transfusing blood type AB into recipients in an effort to avoid these associated problems.

Accordingly, the testing logic underlining the present invention will be readily apparent from examination of Table I. The porous plastic member is "read", to determine whether or not to continue with a reaction, at some point distal to the pick up point in order to permit clear and objective discrimination between the agglutination and nonaggultionation. As will become apparent, various embodiments of the present invention provide for housings totally enclosing the porous plastic member except for appropriately spaced windows in order to view and objectively determine whether transfusion is to continue.

TABLE I

| RECIPIENT IS GIVEN UNIT OF BLOOD KNOWN TO BE TYPE: | RECIPIENT IS TESTED TO CONFIRM HE/SHE IS ACTUALLY TYPE: | UNIT OF BLOOD HAS ABO TEST KIT ATTACHED TO BAG LABELLED: | THE KIT CONTAINS SALINE, POROUS PLASTIC MEMBER AND: | |
|---|---|---|---|---|
| A | A | TYPE "A" KIT | ANTI-"A" | |
| B | B | TYPE "B" KIT | ANTI -"B" | |
| AB | AB | TYPE "AB" KIT | ANTI-"A" & ANTI- "B"[3] | (contd page 9) |
| O | O | TYPE "O" KIT | A PLACEBO[1] | |

[1] IF it was desired to actually test type "O" patient, the following logic would result;

| O | O | TYPE "O" KIT | ANTI-"A" & ANIT-"B" |
|---|---|---|---|

TABLE 1 CONTINUED

| THE ANTIBODY WILL BE USED TO AGGULTINATE RECIPIENT'S RBCs EXPECTED TO BE TYPE: | THE TRANSFUSION CAN CONTINUE IF POROUS PLASTIC MEMBER APPEARS: | THE TRANSFUSION WILL BE STOPPED IF POROUS PLASTIC MEMBER APPEARS: |
|---|---|---|
| A | COLORLESS[2] | RED |
| B | COLORLESS[2] | RED |
| AB | COLORLESS[2] | RED |
| O | COLORLESS[2] | TYPE "O" DONOR BLOOD CAN BE GIVEN TO A, B, AB & O RECIPIENT |
| ANTI-"A" & ANTI -"B" WILL NOT AGGLUTINATE TYPE "O" BLOOD | RED | COLORLESS |

2. If the antisera contains a colored dye (i.e,, green), the colored dye will be visible and serves as a signal to continue with transfusion and that test kit is working.

3. The AB kit actually contains 2 porous plastic members for separate testing of the recipient blood in each of two wells, each well having only anti-A or anti-B respectively.

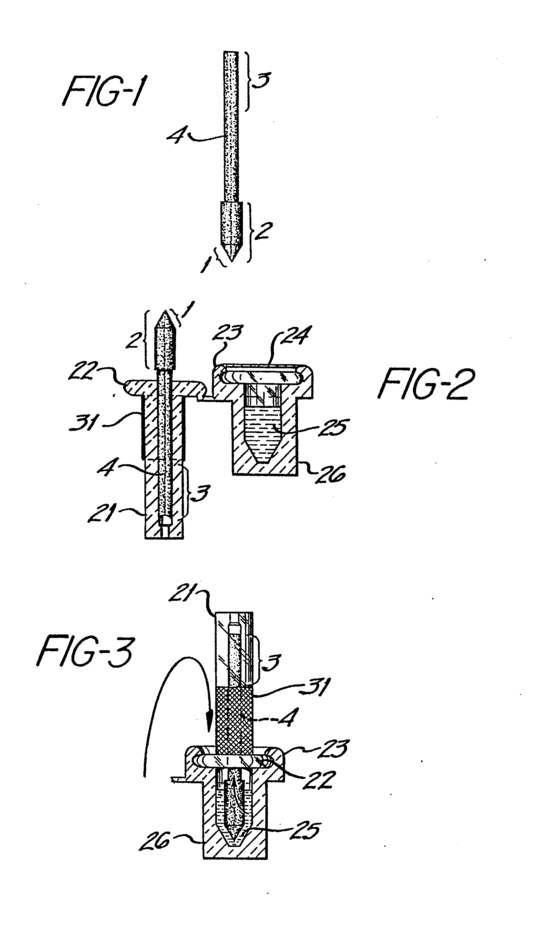

The porous plastic member is preferably molded from polyethylene in a shape such as that shown in Figure 1. Generally, the porous plastic member is elongated to permit capillary action to discriminate between agglutinated cells in reaction area 2 obtained from pick up point 1 to permit fluid travel generally to observation area 3. Cells that agglutinate can be expected to be trapped within the porous structure in reaction area 2 and along the way to observation area 3 so that only the blood serum and antisera diluent arrive in the observation area 3. As noted in Table I, if the antisera is adjusted to contain a color dye, this dye will be visible in observation area 3 in the cases of agglutination. In cases of nonagglutination, the red blood cells will travel to observation area 3 and be clearly visible as a distinct red color. In order to permit the movement of nonagglutinated cells and prevent movement of agglutinated cells, it has been found preferable to employ a porous plastic member having an average pore size of between 10 and 25 microns. This pore size is in reality, an "effective pore size" since the porous plastic is actually a matrix type structure and the term "pore" is somewhat misleading. Pore sizes significantly in excess of 50 microns have been tested and found insufficiently small to adequately inhibit the movement of agglutinated cells.

Further, it has been preferable to employ polyethylene porous plastic members that are additionally treated chemically to make the pore-channel surfaces hydrophilic in order to improve capillary action. Ideally, the porous plastic member is also treated with polyvinylpyrrolidone (PVP) in order to decrease the pick up rate of blood from the finger and thereby improve the agglutination reaction by either providing more reaction time due to decreased travel rates or by direct chemical enhancement. The PVP concentrations found preferable are in the range of approximately 0.5% to 3%. Pick up portion 1 of the porous

ORD-44

plastic member 4 is ideally pointed to facilitate absorption of the patient's blood from a fingertip prick. It has been found preferable to dimension the tip area 2 to hold from 7 to 10 microliters of blood. Further, tip section 1 is preferably conically shaped to ensure a maximum surface area for the antisera to enter the tip and react with the patient's blood. Thus, by proper selection of dimensions and porosity, a porous plastic member 4 can be produced which permits the accurate absorption of a specified volume of patient blood for reaction with antisera and provide a test time of within 30 to 60 seconds.

With reference to Figure 2, an embodiment utilizing the porous plastic member 4 of Figure 1 is shown. The porous member 4 is held within a holder means 21 having locking tabs 22 for engagement within section 23 of chamber housing 26. The chamber housing 26 further incorporates a well of antisera 25 containing the appropriate antisera in accordance with the donor bag blood type. Housing 26 may be further provided with a removable or puncturable cover 24 for maintaining the antisera within the housing, preferably in a sterile condition. The member holder 21 may be removably attached to housing 26 through a breakaway tab 27 in order to facilitate storage. In use, the member holder 21 is broken away from the housing 26, and the tip section 1 of porous member 4 is applied to a drop of the patient's blood until tip portion 2 is filled with blood. At that point, the cover 24 is removed or punctured by the tip as tip section 2 is inserted into antisera bath 25 until locking tabs 22 engage locking area 23. Following an appropriate time, observation area 3 is observed for purposes of detecting whether agglutination has occurred (absence of red, a go ahead condition) or whether there has been no agglutination (presence of red, a stop condition). A preferred embodiment will incorporate a mask covering the portion between observation area 3 and tip section 2 in order to prevent premature observation and to allow sufficient time for the antisera to react and the fluid front to enter observation area 3. Figure 3 shows the relative relationship of the holder 21 to housing 26 following insertion as well as a mask 31 for preventing premature reading.

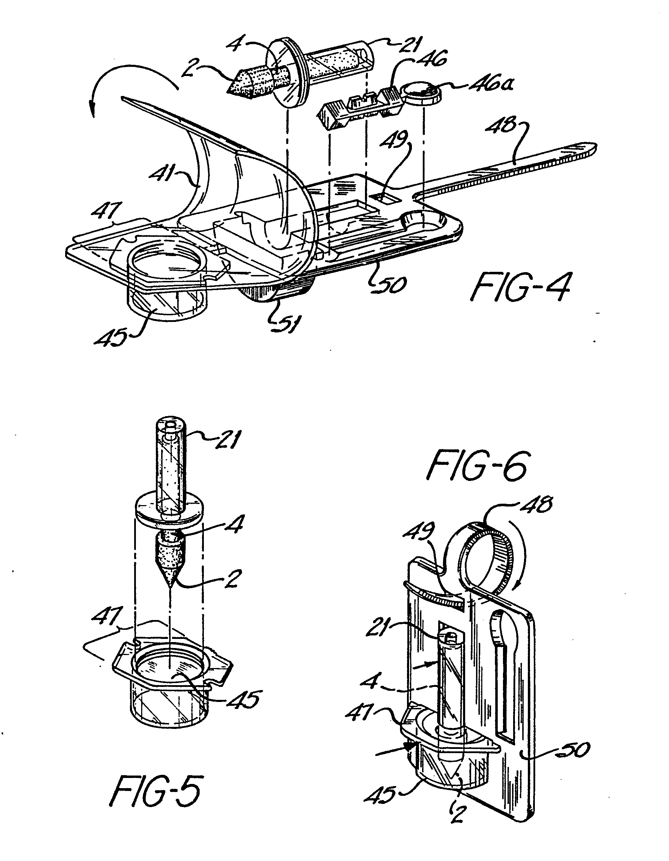

An alternative embodiment of the present invention is depicted in Figures 4, 5 and 6. This embodiment includes a lancet 46 having a breakaway portion 46a for exposing a sharpened sterile pin useful for obtaining the patient's blood in accordance with well-known techniques. This lancet is typically designed to be disposable and one such lancet is available from MONOJECT, Division of Sherwood Medical, St. Louis, Montana 63103. Although the presence of a lancet is an optional feature of the present invention, the preferred embodiment of the test kit will incorporate the lancet in order to promote efficiency and ease of the test. The lancet 46 may be removably held in housing 50 in a convenient location. Similarly, the porous plastic member 4 is held within member holder 21 also removably mounted on housing 50 in a convenient location. Such a mounting may, for instance, be a snap fit or may be held in a cradle by a removable stick on label 41. Section 47 of housing 50 is preferably removable and contains well 45 for the antisera.

Figure 5 demonstrates its removal and the insertion of the porous plastic member. Insertion will preferably be accomplished with a snap fit in order to prevent excess free parts following the test and thereafter, the entire porous member, holder, chamber assembly 4, 21, 47 respectively may be remounted on housing 50 so that chamber housing section 47 is inserted into slot 51 as shown in Figure 6. Observation area on the porous member 4 may be either delimitated on the housing 50 or alternately,

0104881

holder 21 may be masked appropriately. Housing 50 will preferentially include a fastening means such as tie 48 for insertion through slot 49 to aid in attachment of the test kit to the donor bag.

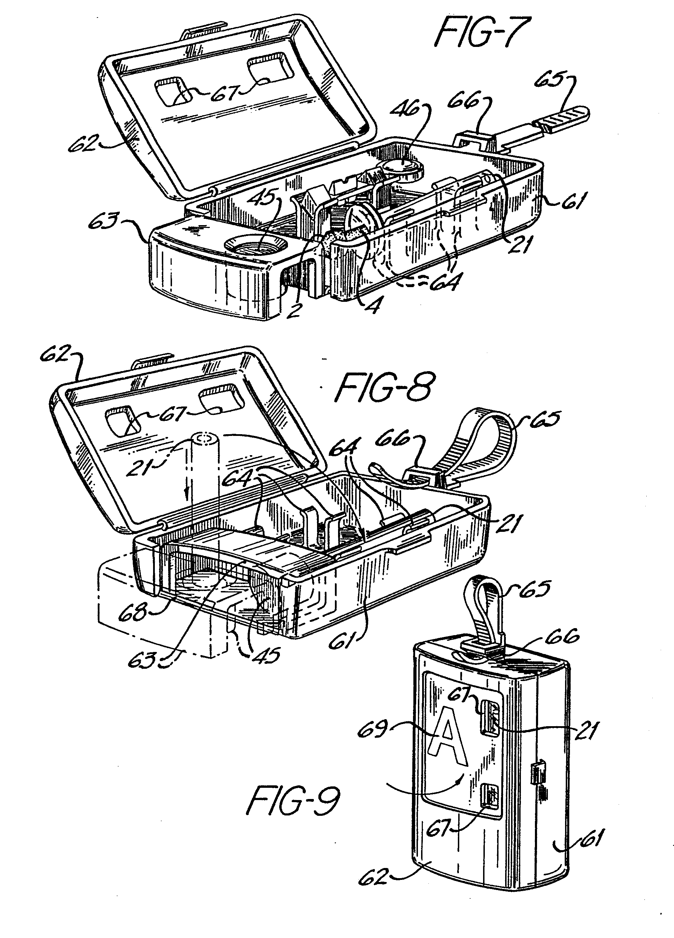

An alternative and most preferred embodiment is depicted in Figures 7, 8 and 9 showing various views and steps in the test kit employment. With reference to Figure 7, there is shown a housing 61 having a door 62 as well as hingable portion 63 containing antisera well 45. The housing 61 has removably mounted therein lancet 46 as well as porous member 4 held in holder means 21. The lancet 46 and porous member/holder 4,21 may be removably mounted by snap fitting support arms 64 as shown in Figure 8. Also shown in Figure 8 is tie strap 65 for engagement with holding means 66 to preferentially permit nonremovable - attachment to the blood donor bag. Figure 8 also shows the intermediate step following insertion of the porous member-tip section 2 into chamber 45 and the swinging of section 63 along hinge 68 to permit remounting of the holder within housing 61 along support arm 64. Figure 9 illustrates the subequent closure of cover 62 with window ports 67 permitting observation of agglutination or nonagglutination reactions. Ideally, cover 62 will include identification means 66 indicating the antisera present within well 45 as well as the type of donor bag with which the test kit is intended to be employed.

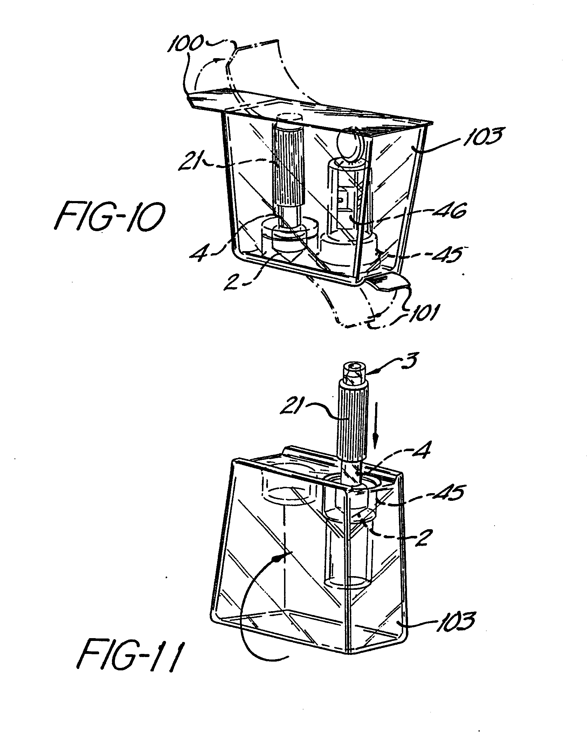

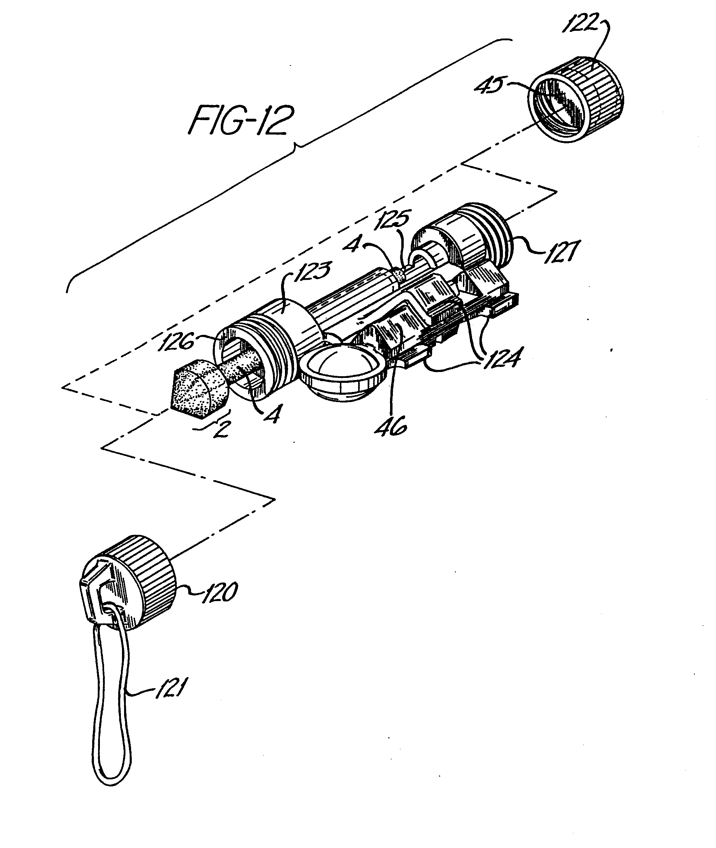

Figures 10 and 11 illustrate an alternative embodiment showing case 103 having peel off label 100 to expose and permit removal of lancet 46 as well as porous plastic member holder 21 for use. Subsequently, removable label 101 is peeled off after inverting the case 103 as shown in Figure 11 to permit insertion of the porpous plastic member tip section into well 45. Observation for the presence or absence of agglutination takes place in observation area 3.

Still another alternative embodiment is shown in Figure 12. Porous plastic member 4 is held within housing 123 and covered prior to test with hanger cap 120. The hanger cap 120 is unscrewed prior to test, lancet 46 is removed from holding arms 124, and section 2 is applied to the recipient's blood as obtained by lancet 46. Cap 122 containing the serum well is unscrewed from housing 123 and the section 2 is inserted therein as cap 122 is screwed onto end 126. Housing 123 is then fastened at end 127 to the hanger cap and the entire device attached to the blood donor bag by tie 121. Presence or absence of agglutination is viewed through observation port 125.

It will be readily apparent, from the descriptions provided above as well as the embodiments illustrated, to one skilled in the art that numerous alternatives are possible, particularly with respect to the design features of the porous plastic member and containing housings, without departing from either the spirit or scope of the present invention.

What is Claimed is:

1. · A bedside kit for testing ABO compatabilty of a transfusion recipient to an A, B, or AB labeled blood unit comprising:

    a) a housing having at least one unsealable chamber containing an effective concentration and volume of antisera reactive with the A and/or B antigen containing blood unit; and

    b) a porous plastic member adapted to pick up an effective volume of blood from the transfusion recipient by capillary action and further adapted to communicate with said antisera in said chamber to permit reaction of said blood with said antisera within said member, said porous member further having a porosity characteristic which permits capillary movement of nonagglutinated red blood cells within said porous member but which does not permit significant movement of agglutinated red blood cells whereby agglutination of recipient's cells as determined by the substantial absence of red blood cell movement serves to indicate blood type incompatibility.

2. The bedside kit as provided in Claim 1 wherein the porous member is adapted to pick up approximately 7-10 microliters of recipient's blood.

3. The bedside kit as provided in Claim 1 wherein the porous member has an effective porosity in the range of approximately 10-50 microns.

4. The bedside kit as provided in Claim 1 wherein the antisera is of sufficient titer to agglutinate AB type red blood cells and has a volume of approximately 50 microliters within said chamber.

5. The bedside kit as provided in Claim 4 wherein the antisera further comprises an effective concentration of a substantially noninterfering dye whereby the dye is drawn through the member by capillary action and becomes visible if there is agglutination but which, in the absence of agglutination, is essentially masked by the presence of red blood cells.

6. The bedside kit as provided in Claim 1 which further comprises porous member holder means adapted to permit the porous member to communicate with the recipient's blood and the antisera in the chamber, and wherein the housing is adapted to removably engage the member holder means.

7. The bedside kit as provided in Claim 6 which further comprises a lancet removably mounted on said housing for obtaining a blood sample from the transfusion recipient.

8. The bedside kit as provided in Claim 6 wherein the housing is adapted for attachment to the blood unit and is further adapted to hold the porous member and porous member holder means for observation of presence or absence of agglutination.

9. The bedside kit as provided in Claim 1 wherein the porous plastic member is made of polyethylene.

10. The bedside kit as provided in Claim 9 wherein the porous member is coated with means for reducing hydrophobicity and wherein the porous member is treated with PVP in the concentration range of about 0.5%-3% for enhancing hemagglutination reactions.

11. A kit for testing the ABO compatability of a transfusion recipient to a labeled blood unit comprising antisera reactive with said labeled blood unit, a lancet for obtaining a blood sample from recipient, a porous plastic member mounted within member holder means and adapted to pick up an effective volume of recipient blood by capillary action and further adapted to communicate with said antisera and said member further having an effective porosity of approximately 10-50 microns whereby nonagglutinated red blood cells move within said porous member by capillary action but wherein agglutinated red blood cells do not undergo significant movement.

12. A reagent kit for attachment to a unit of donor blood to be given a patient comprising means for attaching said kit to said unit of donor blood, antisera specific for at least one antigen present in the donor blood, and means for mixing said antisera with a biological sample from said patient containing blood type antigens.

ORD-44

FIG-1

FIG-2

FIG-3

2/5

4  2  21  46  46a

41  41  49  48

45  51  50  *FIG-4*

21  4  2  41  45  *FIG-5*

*FIG-6*

49  48  21  4  47  45  2  50

FIG-7

67
62
46
66
65
45
63
61
21
2
4
64

FIG-8

62
67
21
64
66
65
64
21
68
63
45
61

69
A
67
21
67
62
65
66
21
61

FIG-9

FIG-10

FIG-11

FIG-12